# EUROPEAN PATENT SPECIFICATION

(11) **EP 0 600 983 B1**
(45) Date of publication and mention of the grant of the patent: **27.03.1996**
(21) Application number: 92917836.6
(22) Date of filing: 05.08.1992
(51) Int. Cl.: C12P 19/62, C12N 1/20

(54) **A FERMENTATION PROCESS FOR PRODUCING NATAMYCIN**
FERMENTATIONSPROZESS ZUR HERSTELLUNG VON NATAMYCIN
PROCEDE DE FERMENTATION POUR LA PRODUCTION DE NATAMYCINE

(30) Priority: 05.08.1991 US 740545
(43) Date of publication of application: 15.06.1994
(73) Proprietor: BIO-TECHNICAL RESOURCES, INC., Manitowoc Wisconsin 54220 (US)
(72) Inventor: EISENSCHINK, Michael, Allen, Manitowoc, WI 54220 (US); MILLIS, James, R., Kohler, WI 53044 (US); OLSON, Phillip, Terry, Manitowoc, WI 54220 (US)
(74) Representative: Woodcraft, David Charles
(86) International application number: US9206323
(87) International publication number: WO9303170

(56) References cited:
- CH-A- 359 517
- GB-A- 846 933

## Description

The present invention relates to a process for producing natamycin characterized by inoculum preparation, inoculum propagation and fermentation.

Natamycin is a member of the polyene family of antimycotics. The compound natamycin is a tetraene with a molecular weight of about 666, empirical formula corresponding generally to C₃₃H₄₇NO₁₃. and it contains a glycosidically-linked carbohydrate moiety, mycosamine. Natamycin (also called pimaricin) has an isoelectric point of about pH 6.5, and the structure exists typically in two configurations: the enol-structure and the keto-structure.

A conventional fermentation process for producing natamycin is described in American Cyanamid's British Patent No. 846,933 (1960).

Despite the antibiotic and anti-fungal value of natamycin, very little commercial use has been made of this product. One major reason for the limited use is a prohibitively high manufacturing cost.

It is an object of the present invention to overcome the inefficiencies of conventional processes and provide a process for producing useful quantities of natamycin in a cost-effective manner by propagating and fermenting an inoculum in predetermined media.

### SUMMARY OF THE INVENTION

The present invention relates to fermentation by an organism capable of producing natamycin. Particularly the present invention is directed to preparing (e.g. sporulation) and propagating an inoculum comprising a Streptomyces species that, during fermentation, produces natamycin. The Streptomyces species is exposed to a series of predetermined environments and/or mediums which improve the rate at which natamycin is produced.

By providing an improved environment for the Streptomyces species the yield of natamycin may be increased to values in the range of about 5 through at least about 12 g/liter. It has been discovered that greatly increased yields of natamycin can be obtained in a fermentation process by using the following inoculum propagation and fermentation media:
1. A suitable medium for inoculum propagation comprises:
   (a) a protein nitrogen source, i.e. a nitrogen source in the form of a protein, in an amount of from about 2-16 g/l, normally about 8 g/l; and
   (b) a metabolizable carbon source present in an amount which is sufficient to avoid total carbon depletion, usually 5-30 g/l of medium, and normally about 15 g/l;
2. A suitable medium used during fermentation to induce the inoculum to produce natamycin comprises:
   (a) about 80-250 g/l of a metabolizable carbon source; and
   (b) a protein nitrogen source containing a high level of protein and trace ingredients.

The protein nitrogen source typically comprises a non-yeast protein nitrogen component and a yeast protein nitrogen component. These two protein nitrogen components are desirably present in a ratio ranging, respectively, from about 5:1 to 11:1 based on protein contents, and for best results about 8:1.

Usage of media possessing the above characteristics is a key aspect of the present invention which permits the *Streptomyces* species to produce natamycin in a cost-effective manner.

### BRIEF DESCRIPTION OF THE DRAWINGS

Fig. 1 is a schematic block diagram of the process which may be used in the invention for inoculum propagation.

Fig. 2 is a graphical representation of the three phases which occur during fermentation.

### DETAILED DESCRIPTION OF THE INVENTION

In accordance with the present invention, an organism capable of producing natamycin is placed into contact with a predetermined medium to produce an inoculum, and then into a predetermined fermentation production medium that will support maximum metabolic activity of the organism during further propagation and natamycin producing fermentation. During fermentation the organism enzymatically transforms at least a portion of the predetermined production medium into natamycin.

Any organism which comprises a natamycin producing *Streptomyces* species can be used in accordance with the invention. A preferred *Streptomyces* species comprises *Streptomyces gilvosporeus* which has been deposited previously with the American Type Culture Collection (ATCC) in Rockville, Maryland, United States of America, that is registered as ATCC No. 13326.

An inoculum is prepared from a spore suspension of the appropriate spores. The inoculum of the appropriate *Streptomyces* species is subsequently fermented which produces high yields of natamycin when placed into the fermentation medium of the invention. The inoculum is typically exposed to a series of propagation steps wherein each step increases the quantity of the natamycin producing *Streptomyces* cells. After the quantity of *Streptomyces* cells is adequate, the *Streptomyces* is exposed to an environment and/or a medium which is designed to enhance natamycin production when the *Streptomyces* species ferments.

### Spore Suspension

The inoculum is started by collecting the spores of a natamycin producing Streptomyces species which was obtained from the American Type Culture Collection. The spores are germinated to produce an actively growing culture of the *Streptomyces* species. A sterilized (e.g., autoclaved), agar slant is inoculated heavily with the actively growing culture of the *Streptomyces* species(e.g., *Streptomyces gilvosporeus* or any other natamycin producing species), and incubated until the slant is covered substantially entirely with spores. The spores on the agar slant are scraped into a small amount of a liquid, such as water (e.g., distilled water), nutrient medium, etc., to produce an aqueous spore suspension. The resulting spore suspension is propagated to produce the inoculum for the fermentation operation (i.e., natamycin production). For achieving the best results, the spore suspension used to begin inoculum propagation should contain a spore concentration of about 10⁵-10¹⁰ CFU/ml, and, normally, at least about 10⁸ CFU/ml.

A number of agar slant media can be used to promote sporulation of the culture of the *Streptomyces* species (e.g., S. *gilvosporeus),* which will be used to form the spore suspension. Appropriate agar slant mediums typically comprise at least one member of the following group: yeast malt agar, Hickey-Turner agar, GYA agar, Pridham agar, potato dextrose, Bennett's agar, etc.

A high concentration (e.g., 10⁸ CFU/ml), of viable spores within the spore suspension is a key aspect of the present invention. First, if the concentration of spores is too low, it takes much longer to obtain, through inoculum propagation, the quantity of *Streptomyces* cells sufficient for cost-effective natamycin fermentation production. Second, a reduced quantity of spores within the suspension lengthens the total inoculum propagation time and increases the likelihood of a contamination(e.g., by an unwanted organism). Further, a low spore concentration within the suspension may tend to promote the formation of large, tightly packed mycelial pellets. These pellets are unsuitable for obtaining high yields of natamycin due to problems associated with oxygen transfer, mass transfer of nutrients into the pellets, etc. Should the size of mycelial pellets become undesirable, the pellets can be broken apart physically, such as by using a shear force (e.g.,blending).

### Inoculum Propagation

The aqueous spore suspension (e.g., *S*. *gilvosporeus),* discussed above, is germinated and cell multiplication continued until the number of organisms is adequate to be used as an aqueous inoculum for fermentation production of natamycin. A suitable inoculum cell density comprises a dry cell weight of about 1-5 g/l and is used at a volume of about 0.1-10% of the natamycin production medium volume.

The aqueous medium used for inoculum propagation determines the cell density and the metabolic state of the inoculum (e.g., an adequate density of healthy cells is desirable). A sufficient amount of protein nitrogen which contains complex growth factors (e.g., vitamins), inorganic elements (e.g.,potassium,sodium,calcium,etc.), and trace elements(e.g., boron, cobalt, iron, copper, zinc, etc.), that are commonly present in the protein nitrogen source are needed to achieve an inoculum possessing the desired cell density and metabolic state. The protein nitrogen source may be any source that will propagate the spore suspension into an inoculum that will produce the desired high yields of natamycin.

A metabolizable source of carbon must also be supplied to the aqueous inoculum medium in an amount which is sufficient to achieve the desired inoculum cell density. For best results, the carbon source should not be depleted completely during the inoculum propagation. Depletion of the carbon source tends to alter adversely the metabolic state of the inoculum, which may lead to reduced yields of natamycin during fermentation.

Although a variety of aqueous inoculum media can be used effectively in accordance with the present invention, to obtain high yields of natamycin it is advantageous to use predetermined amounts of medium ingredients.

A suitable medium for inoculum propagation may be prepared in water (e.g., low mineral content water, distilled water, etc.), and comprises:
a) a protein nitrogen source in an amount from about 2-16 g/l, normally about 8 g/l; and
b) a metabolizable carbon source present in an amount which is sufficient to avoid total carbon depletion, usually 5-30 g/l of medium, and normally about 15 g/l.

Two specific compositions of a medium appropriate for inoculum propagation are given below.

| Composition 1 | Quantity |
|---|---|
| Difco "Bacto" peptone | 5 g/l |
| Corn steep liquor | 3 g/l |
| Sodium chloride | 10 g/l |
| Glucose | 15 g/l |

| Composition 2 | Quantity |
|---|---|
| Hormel peptone PSR 5 | 8 g/l |
| Sodium chloride | 10 g/l |
| Glucose | 15 g/l |

The inoculum medium which provides nutrients that enhances the production rate of *Streptomyces* cells may be prepared by conventional techniques(e.g., separate or simultaneous sterilization of the carbon and nitrogen sources at temperatures of about 120-140°C). The inoculum medium after sterilization, desirably has a pH of about 7. The spore suspension is introduced to the inoculum medium and the inoculum medium is heated to a temperature of about 25 - 40°C and, normally, about 28 - 35°C.

In order to achieve the large volumes of aqueous inoculum which are desirable for fermentation production of natamycin, several inoculum propagation steps are required, each carried out in a volume greater than the previous step. For example, the inoculum propagation may be conducted in a manner which achieves an exponential increase in the quantity of cells. Particularly, it is advantageous to keep the culture in an exponential growth mode during propagation by effectively increasing the volume of the inoculum during each step of the propagation. This can be done by either minimizing the duration of each step or by minimizing the number of steps. For example, once a predetermined cell density of inoculum has been achieved, the inoculum is transferred to a larger environment(e.g., vessel), for further propagation. By effectively controlling the inoculum propagation a minimum of time and expense is devoted to inoculum propagation and, accordingly, cost-effective natamycin yields during fermentation are increased.

The length of time an individual step in the series of inoculum propagation steps is permitted to continue depends upon the composition of the medium, quantity of *Streptomyces* cells desired, temperature, etc. Typically, an individual propagation step is conducted for about 6 through about at least about 24 hours.

The inoculum propagation process requires aeration of the inoculum. For example, the vessel or flask housing the inoculum, may be agitated on a rotary shaker at about 200 rpm. In one aspect of the invention, the inoculum may be agitated by an impeller which is located within the vessel that houses the inoculum while sterile air is forced into the bottom of the vessel.

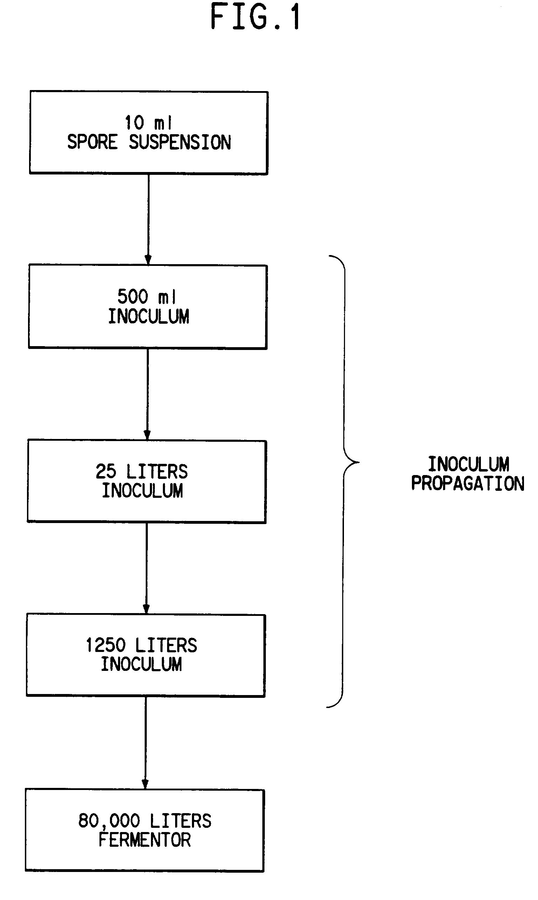

Now referring to Figure 1, this figure is a schematic of the process which may be used to produce the inoculum that is fermented to produce nataymcin. Figure 1 illustrates the volumetric increases in the inoculum which are achieved by propagation that are necessary to obtain a quantity of inoculum that is adequate to produce natamycin in a cost-effective manner. For example, the volume of inoculum is increased from 25 liters to 1250 liters by adding the 25 liters of inoculum to a vessel containing 1225 liters of an aqueous inoculum medium.

### Natamycin Production

Natamycin production is conducted in a fermentation vessel which is capable of housing the fermentation process. One element important for achieving maximum yields of natamycin is the composition of the aqueous fermentation medium. The fermentation medium must contain the proper amounts of metabolizable carbon and protein nitrogen. Also, it is desirable that the medium contains complex growth factors (e.g., vitamins), and inorganic elements (e.g., potassium, sodium, calcium, etc.), and trace elements (e.g., boron, cobalt, iron, copper, zinc, etc.), that are commonly present in the protein nitrogen source.

A suitable medium for fermentation may be prepared in water(e.g., low mineral content tap water, distilled water, etc.), and comprises:
a) about 80-250 g/l of a metabolizable carbon source; and
b) at least 15 g/l and, normally about 20 g/l through 80 g/l, of a protein nitrogen source containing a high level of protein and trace ingredients. The protein nitrogen source may comprise a non-yeast protein nitrogen component and a yeast protein nitrogen component. These two protein nitrogen components are usually present in the ratio ranging, respectively, from about 5:1 to 11:1 based on protein contents, and for best results generally about 8:1.

The protein nitrogen source may be supplied from a wide range of sources. For example, soy protein products(e.g., isolates, flours, meals, etc.), may comprise the non-yeast protein nitrogen source (e.g., desirable natamycin yields are obtained with a soy protein source comprising 80-95% protein). The protein nitrogen may also comprise beef extract, protein hydrolysates (e.g.,peptones), and/or yeast (e.g., extracts, autolysates, etc.).

As discussed above, the production medium must also include a source of carbon which is metabolizable by the *Streptomyces* species. The carbon source may supplied in any expedient form such as glucose, polysaccharide, corn and potato starches, etc.

Moreover, in one aspect of the invention, it is not necessary to initially introduce the entire quantity of the carbon source which is required to produce natamycin, as a starting component of the natamycin production medium (e.g., the initial quantity of the carbon source is not adequate for complete fermentation). In this aspect of the present invention, carbon source addition may be performed during the natamycin production so as to maintain a quantity of carbon source of about 5-30 g/l, and usually 20 g/l. Thus, an appropriate quantity of a suitable carbon source is added to the fermentation medium either initially and/or added after the fermentation has begun. For example, the carbon source may be present in the fermentation medium in an amount of about 40-100 g/l. Thereafter, during the major period of fermentation, carbon source is continually added to the fermentor at a rate which is at least equivalent to the rate at which the carbon source is consumed by the *Streptomyces* species during the fermentation process(e.g., to maintain the carbon source concentration at or above a minimum level). Toward the end of the fermentation process and after the major fermentation period, the carbon source addition is discontinued so that little or no carbon source is left at the end of the fermentation cycle (e.g., the quantity of the carbon source substantially equates to the particular quantity of carbon source within the fermentation medium which is necessary to complete the fermentation process).

The natamycin production medium, which provides nutrients for the *Streptomyces* fermentation and natamycin production is prepared by conventional techniques (e.g., separate or simultaneous sterilization of the carbon and nitrogen sources at temperatures of about 120-140°C). The production medium, after sterilization, desirably has a pH of about 7.

The inoculum is introduced into a fermentation vessel until a concentration of about to 0.1-10%, usually about 2%, by volume is achieved in the production medium(e.g., the quantity of inoculum may be sufficient to inoculate a plurality of fermentors). The remainder of the volume of the fermentor comprises the fermentation medium. Any technique is acceptable for introducing the inoculum to the production medium within the fermentor which delivers the inoculum in an active metabolic state.

The fermentation or production medium is brought to a temperature of about 25°-40°C, and normally 28°-35°C. The length of time which the fermentation process is allowed to continue depends upon the composition of the fermentation medium, temperature, quantity of *Streptomyces* cells in the inoculum, quantity of natamycin desired, etc. Typically, the fermentation process is conducted for about 70 through at least about 168 hours.

Oxygen is supplied to the natamycin production medium during fermentation. It is advantageous to maintain a dissolved oxygen level in the production medium of about 20%-80% of air saturation during the major portion of the fermentation. The ability to achieve a suitable dissolved oxygen level may be enhanced by proper coordination of the aeration and/or agitation rate. For example, the fermentation or production medium is aerated by forcing air(e.g., sterile air), through the fermentation medium, usually at a rate of about 0.3 through at least about 1.0 volumes of air per volume of fermentation medium. In one aspect of the invention it is desirable to agitate the fermentation medium while being aereated. Further, the rate of aeratation may be sufficient to cause agitation of the fermentation medium.

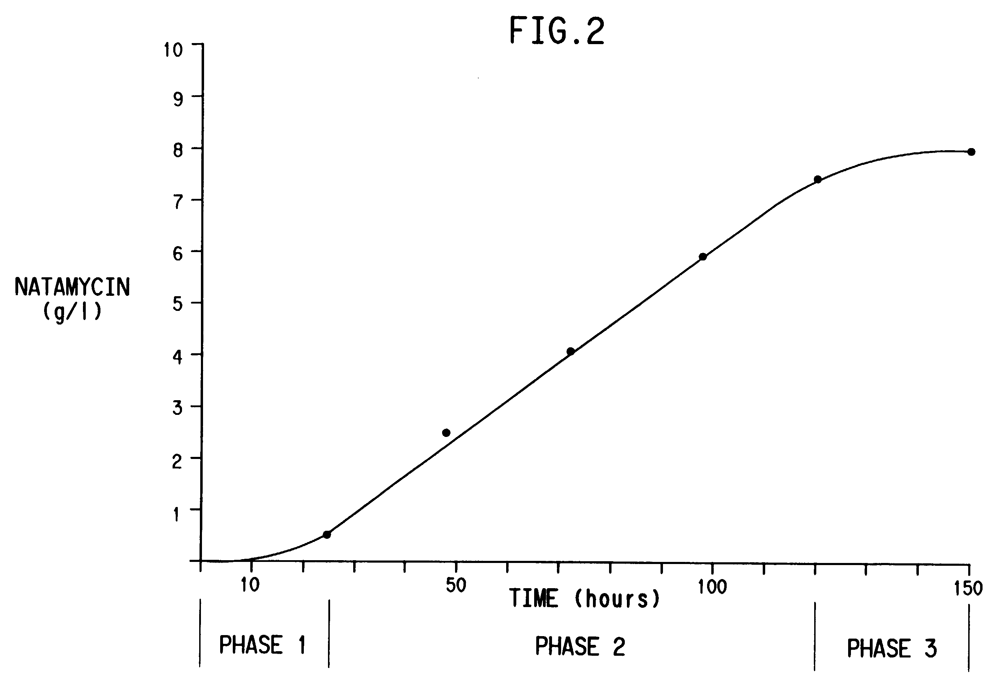

Referring now to Fig. 2, the relationship between time and nataymcin concentration is shown for each of the three phases of the process. The first phase includes addition of the carbon source to the fermentation medium and growth or multiplication of the *Streptomyces* species. The first phase is also accompanied by natamycin production. The concentration of natamycin in the fermentation broth increases as the progagation of the cells of the *Streptomyces* species increases. The increase in the concentration of natamycin increases generally exponentially with time during the first phase. Eventually, the concentration of natamycin will increase constantly with time, which indicates that the second phase (i.e., the major phase) of natamycin production as been achieved. The third phase is characterized by a plateau in the concentration of natamycin (e.g., which may be due to a slowing of the metabolic activities of the *Streptomyces* species). The concentration of natamycin within the fermenter may be analyzed with respect to time in order to ascertain the current phase of fermentation. It is desirable to use a medium and/or an environment which induces the second phase of fermentation to be rapidly reached and maintained in order to maximize the overall quantity of natamycin that is produced.

In one aspect of the invention, it may be desirable to add an anti-foaming agent (e.g. a silicone de-foamer), to the fermentation medium in an amount of from about 0.01%-1% by volume of the fermentation or natamycin production medium when it is desirable to control foaming.

The natamycin production medium after inoculum addition has a pH of about 7.0, which decreases slowly during fermentation to about 4.5. The lowered pH is a result of the metabolic activities of the *Streptomyces* species. After production of natamycin is completed, depending upon the end-use of the fermentation broth, it may be desirable for the fermentation medium to have a lowered pH since the fermentation broth is more readily processed(e.g., at a higher pH of about 7 the fermentation broth may become relatively viscous, and recovery of natamycin from the fermentation broth may be more difficult).

When the present invention is practiced appropriately (e.g., effective handling of the *Streptomyces* inoculum, selection of media, etc.), the resultant fermentation broth will normally include at least about 5 g/l of natamycin. In certain cases, the level of natamycin production may range from about 7 g/l through at least about 12 g/l.

The natamycin can be separated from the production medium. In certain cases, the natamycin may be extracted from the fermentation broth and crystallized. Examples of acceptable techniques for obtaining crystalline natamycin can be found in U.K. Patent No. 846,933.

The invention is demonstrated by the following Example which is intended to illustrate, not limit, the scope of the contemplated equivalents. Unless specified otherwise, commercially available reagent grade materials were used to conduct the following Example.

### EXAMPLE

In the following tests, agar slants of the following compositions are prepared using distilled water.
3 g/l yeast extract (Difco "Bacto" Yeast Extract)
3 g/l malt extract (Difco Malt Extract)
5 g/l peptone (Difco "Bacto" peptone)
10 g/l glucose
15 g/l agar.
The agar was sterilized at about 121°C for about 15 minutes.

An inoculum medium of the following composition was prepared in distilled water, and the pH was adjusted to about 7.0 with potassium hydroxide.
20 g/l glucose
10 g/l sodium chloride
6 g/l corn steep liquor (PPM (brand), Corn Steep Liquid)
6 g/l peptone (Difco "Bacto" peptone)
The inoculum medium was sterilized at about 121°C for about 15 minutes.

*Streptomyces gilvosporeus,* American Type Culture Collection Registration No. 13326, was obtained from the American Type Culture Collection as a freeze-dried spore suspension and used as the culture source. The culture was held on the agar slants at about 25°C until the culture sporulated.

The agar slants sporulated heavily within about 10 days and were used after 10-20 days. Spores were scraped off these agar slants into the inoculum medium to achieve a spore suspension concentration of about 10⁸ CFU/ml. About 2 ml of the spore suspension was added to about 100 ml of the inoculum medium in a 500 ml baffled flask. The inoculum in the baffled flask was incubated for about 48 hours at about 29°C and agitated at about 200 rpm on a rotary shaker. After about 48 hours about 4 ml of this culture was added to about 200 ml of inoculum medium in a 1000 ml baffled flask, to propagate the inoculum. This inoculum was then incubated for about an additional 24 hours at about 29°C and agitated at about 200 rpm on a rotary shaker. The inoculum thus produced was used to inoculate 8 l of production medium.

The natamycin production medium used in this Example was of the following initial composition:
19.5 g/l soy protein isolate (ADM, "Profam" S970)
4.5 g/l yeast extract (Stauffer, Type KAT)
0.2 ml defoamer (Mazu, DF 289)
The production medium was prepared in distilled water in a 14 1 fermenter and the pH was adjusted to about 7.6 with potassium hydroxide. The fermenter was then sterilized for about 15 minutes at about 121°C. Glucose was sterilized separately as a 50% solution in distilled water.

Before inoculation, the production medium was heated to about 29°C and the glucose was added to achieve an initial concentration of glucose of about 40 g/l. An aeration rate of about 0.3 v/v-min. (volumes of air per volume of medium per minute) and an agitation rate of about 300 rpm was established for the fermentor.

The inoculum discussed above containing *Streptomyces gilvosporeus,* (ATCC Registration No. 13326), was added to the fermentation vessel until the fermentation vessel had an inoculum content of about 2% by volume. Glucose was added to the inoculum after about 40 hours of fermentation in order to maintain a glucose concentration of about 20 g/l glucose in the fermentation vessel. This was done by feeding glucose to the fermenting vessel at a rate of about 1 g/l-hr. The agitation rate of the fermentation vessel was increased as necessary to maintain a dissolved oxygen level of about 50% of air saturation.

### Test #1 -

Proceeding as described above, starting with an initial volume of about 8.0 1 production medium and continuing the fermentation cycle time for about 117 hours, with a total glucose addition of about 110 g/l, a yield of 7.3 g/l natamycin in 8.7 1 of fermentation broth was obtained (64 g natamycin total).

### Test #2 -

The procedure of Test #1 was repeated with the following exception: a sparsely sporulated agar slant was used to prepare the spore suspension and, as a result, the spore concentration in the initial spore suspension was only about 5x10⁴ CFU/ml. The yield of natamycin was reduced to about 5.2 g/l in 8.7 l of fermentation broth (45 g natamycin total).

### Test #3 -

The procedure of Test #1 was repeated with the following exception: the fermentation medium was changed to about 10 g/l glucose, about 2 g/l beef extract, about 2 g/l yeast extract, about 0.5 g/l asparagine and about 0.5 g/l dibasic potassium phosphate. No glucose was added subsequently. After about 72 hours of production, the yield of natamycin was about 0.75 g/l in about 0.1 l of fermentation broth.

### Test #4 -

Proceeding as described in Test #1, but using Pridham agar for sporulation and only about 12 hours incubation for the initial inoculum propagation step, and using a medium of 6 g/l yeast extract and 26 g/l soy protein isolate, and adding 185 g/l glucose over a 106 hour fermentation cycle, there was obtained a natamycin yield of about 10.1 g/l in about 8.4 l of fermentation broth (85 g natamycin total).

A review of Tests 1 through 4 in this Example illustrates that an increase in the concentration of the spores in the inoculum and the nutrient content within the fermentation medium tends to increase the overall quantity of natamycin which may be recovered.

## Claims

1. A process of preparing natamycin by fermentation wherein:
(a) the inoculum comprises a natamycin-producing Streptomyces culture; and a medium comprising protein nitrogen and a metabolizable carbon source in excess of that required to avoid carbon depletion during inoculum propagation; and
(b) the natamycin production medium comprising 80 to 250 g/l of a metabolizable carbon source, and 15 to 80 g/l of a nitrogen protein source.

2. The process of claim 1 wherein the metabolizable carbon source is initially present in the natamycin production medium in a concentration of 40 ∼ 100 g/l and wherein sufficient further carbon source is added to the production medium during fermentation so as to maintain the carbon source concentration above a minimum level during the major fermentation period.

3. The process of claim 1 or 2 wherein carbon source is added to the natamycin production medium so as to maintain the carbon source concentration at 5 to 30 g/l.

4. The process of any one of the preceding claims wherein said culture is Streptomyces gilvosporeus ATCC 13326.

5. The process of any one of the preceding claims wherein the inoculum medium contains 2 to 16 g/l of a protein nitrogen source and 5 to 30 g/l of a metabolizable carbon source.

6. The process of any one of the preceding claims wherein said spore suspension contains a spore concentration of 10⁵-10¹⁰ CFU/ml.

7. The process of any one of the preceding claims wherein said production medium contains non-yeast and yeast protein nitrogen components, said non-yeast and yeast components being present in the ratio range respectively of 5:1 to 11:1 based on protein contents.

8. The process of claim 7 wherein said ratio is about 8:1.

9. The process of claim 7 or 8 wherein said non-yeast component is a soy protein nitrogen source.

10. The process of any one of the preceding claims wherein at least 7g of natamycin is produced per liter of natamycin production medium.

## Patentansprüche

1. Verfahren zur Herstellung von Natamycin durch Fermentation, bei dem:
(a) das Inoculum eine Natamycin-reduzierende Streptomyces-Kultur und ein Medium umfaßt, das Protein-Stickstoff und eine metabolisierbare Kohlenstoffquelle im Überschuß derjenigen, die erforderlich ist, um eine Kohlenstoff-Verarmung während der Inoculum-Propagation zu vermeiden, umfaßt, und
(b) das Natamycin-Produktionsmedium 80 bis 250 g/l metabolisierbare Kohlenstoffquelle und 15 bis 80 g/l Stickstoffproteinquelle umfaßt.

2. Verfahren nach Anspruch 1, bei dem die metabolisierbare Kohlenstoffquelle zu Beginn in dem Natamycin-Produktionsmedium in einer Konzentration von 40 bis 100 g/l vorhanden ist und worin dem Produktionsmedium während der Fermentation noch genug Kohlenstoffquelle zugesetzt wird, so daß die Kohlenstoffquellen-Konzentration während des Hauptfermentationszeitraums oberhalb eines Minimalniveaus gehalten wird.

3. Verfahren nach Anspruch 1 oder 2, bei dem die Kohlenstoffquelle dem Natamycin-Produktionsmedium zugesetzt wird, so daß die Kohlenstoffquellen-Konzentration bei 5 bis 30 g/l eingehalten wird.

4. Verfahren nach einem der vorhergehenden Ansprüche, bei dem die Kultur Streptomyces gilvosporeus ATCC 13326 ist.

5. Verfahren nach einem der vorhergehenden Ansprüche, bei dem das Inoculummedium 2 bis 16 g/l einer Proteinstickstoffquelle und 5 bis 30 g/l einer metabolisierbaren Kohlenstoffquelle enthält.

6. Verfahren nach einem der vorhergehenden Ansprüche, bei dem die genannte Sporensuspension eine Sporenkonzentration von 10⁵-10¹⁰ CFU/ml enthält.

7. Verfahren nach einem der vorhergehenden Ansprüche, bei dem das Produktionsmedium Nicht-Hefe- und Hefeproteinstickstoffkomponenten enthält, wobei die Nicht-Hefe- und Hefekomponenten in einem Verhältnisbereich von 5:1 bis 11:1, bezogen auf den Proteingehalt, vorhanden sind.

8. Verfahren nach Anspruch 7, bei dem das Verhältnis etwa 8:1 beträgt.

9. Verfahren nach Anspruch 7 oder 8, bei dem die Nicht-Hefekomponente eine Sojaproteinstickstoffquelle ist.

10. Verfahren nach einem der vorhergehenden Ansprüche, bei wenigstens 7 g Natamycin pro 1 Natamycin-Produktionsmedium produziert werden.

## Revendications

1. Un procédé de production de natamycine par fermentation, dans lequel :
(a) l'inoculum comprend une culture de *Streptomyces* producteur de natamycine ; et un milieu comprenant de l'azote protéique et une source de carbone métabolisable en excès de ce qui est nécessaire pour éviter un épuisement du carbone pendant le développement de l'inoculum ; et
(b) le milieu de production de natamycine comprend 80 à 250 g/ℓ d'une source de carbone métabolisable et 15 à 80 g/ℓ d'une source protéique d'azote.

2. Le procédé de la revendication 1, dans lequel la source de carbone métabolisable est initialement présente dans le milieu de production de la natamycine à une concentration de 40 à 100 g/ℓ et dans lequel on ajoute encore suffisamment de source de carbone au milieu de production pendant la fermentation de manière à maintenir la concentration de la source de carbone au-dessus d'une valeur minimale pendant la principale période de fermentation.

3. Le procédé de la revendication 1 ou 2, dans lequel on ajoute la source de carbone au milieu de production de la natamycine de manière à maintenir la concentration de la source de carbone entre 5 et 30 g/ℓ.

4. Le procédé de l'une quelconque des revendications précédentes, dans lequel ladite culture est une culture de *Streptomyces gilvosporeus* ATCC 13326.

5. Le procédé de l'une quelconque des revendications précédentes, dans lequel le milieu d'inoculum contient 2 à 16 g/ℓ d'une source d'azote protéique et 5 à 30 g/ℓ d'une source de carbone métabolisable.

6. Le procédé de l'une quelconque des revendications précédentes, dans lequel ladite suspension de spores a une concentration de spores de 10⁵ à 10¹⁰ UFC/ml.

7. Le procédé de l'une quelconque des revendications précédentes, dans lequel ledit milieu de production contient des composants d'azote protéique non dérivé de levure et dérivé de levure, lesdits composants non dérivé de levure et dérivé de levure étant présents en un rapport compris dans l'intervalle de 5:1 à 11:1 respectivement sur la base des teneurs en protéine.

8. Le procédé de la revendication 7, dans lequel ledit rapport est d'environ 8:1.

9. Le procédé de la revendication 7 ou 8, dans lequel ledit composant non dérivé de levure est une source d'azote protéique de soja.

10. Le procédé de l'une quelconque des revendications précédentes, dans lequel au moins 7 g de natamycine sont produits par litre de milieu de production de natamycine.
